# EUROPÄISCHE PATENTSCHRIFT

(11) **EP 3 654 858 B1**
(45) Veröffentlichungstag und Bekanntmachung des Hinweises auf die Patenterteilung: **21.12.2022**
(21) Anmeldenummer: 18730714.5
(22) Anmeldetag: 06.06.2018
(51) Int. Cl.: A61B 17/3207, A61M 60/135, A61M 60/237, A61M 60/31, A61M 60/523, A61M 60/531, A61B 17/00

(54) **VORRICHTUNG ZUR ZERKLEINERUNG VON ZIRKULIERENDEN TUMORZELLCLUSTERN**
DEVICE FOR THE COMMINUTION OF CIRCULATING TUMOUR CELL CLUSTERS
DISPOSITIF POUR DÉSAGRÉGER DES AGRÉGATS DE CELLULES TUMORALES CIRCULANTES

(30) Priorität: 17.07.2017 WO PCT/EP2017/068024
(43) Veröffentlichungstag der Anmeldung: 27.05.2020
(73) Patentinhaber: Griesmühle Kleinkraftwerk GmbH, 4111 Walding (AT); Universität Basel, 4001 Basel (CH); Johannes Kepler Universität Linz, 4040 Linz (AT)
(72) Erfinder: ACETO, Nicola, 4153 Reinach (CH); BAUMGARTNER, Werner, 4040 Linz (AT); PRIESNER, Kurt, 4111 Walding (AT)
(74) Vertreter: KLIMENT & HENHAPEL
(86) Internationale Anmeldenummer: PCT/EP2018/064935
(87) Internationale Veröffentlichungsnummer: WO 2019/015854

(56) Entgegenhaltungen:
- DE-T2- 69 535 694
- US-A1- 2016 331 378

## Beschreibung

Die vorliegende Erfindung betrifft eine Vorrichtung zur Zerkleinerung oder Inaktivierung von zirkulierenden Tumorzellen (CTC) oder Tumorzellclustern (CTC-Cluster, CTCC) aus einem von einem Tumor betroffenen Organ oder Organteil. Die erfindungsgemäße Vorrichtung dient dabei der Verminderung des Risikos der Metastasenbildung bei Krebserkrankungen.

Wenn im Folgenden der Einfachheit und Verständlichkeit halber der Begriff "Tumor" verwendet wird, dann sind damit auch alle anderen Krebserkrankungen von Organen des menschlichen oder tierischen Körpers gemeint, auch wenn diese sich nicht als umschriebener fester (solider) Tumor ausbilden.

Es ist bekannt, dass bei Krebserkrankungen primäre Tumore Krebszellen absondern und die so abgesonderten Krebszellen in die Blutbahn gelangen und im erkrankten Körper zirkulieren. Diese Krebszellen werden im Folgenden als zirkulierende Tumorzellen, oder auch als CTCs (circulating tumor cells) bezeichnet. Per Definition handelt es sich dabei um Zellen des Primärtumors oder aus Metastasen, die den Zellverband verlassen haben und sich nun in der Blutzirkulation befinden. Die so über die Blutbahn im Körper zirkulierenden Tumorzellen können sich nun wiederum in anderem Gewebe festsetzen und dort zur Bildung von Sekundärtumoren, sogenannten Metastasen, führen. Diese Metastasenbildung ist ein großes Problem bei Krebserkrankungen. In den weit überwiegenden Fällen von Krebserkrankungen mit tödlichem Verlauf ist nicht der Primärtumor kausal für den Tod des Patienten, sondern die Metastasen.

Derzeitige Behandlungsmethoden zielen dabei darauf ab den Primärtumor zu zerstören, um auf diese Weise auch ein Ausstreuen von zirkulierenden Tumorzellen zu unterdrücken und damit das Risiko der Metastasenbildung zu verringern. Wird der Primärtumor operativ in einem chirurgischen Eingriff entfernt, besteht bei einem solchen Eingriff aber stets das Risiko einer Verletzung des Tumorgewebes sowie der im Tumorgewebe und dem umgebenden Gewebe vorhandenen Blutgefäße mit einer entsprechenden Freisetzung von Tumorzellen in den Blutkreislauf und einer damit verbundenen Erhöhung des Risikos der Metastasenbildung.

Untersuchungen an menschlichen Krebspatienten legen nahe, dass bei fortgeschrittener Krebserkrankung die im Blut zirkulierenden Tumorzellen nach wie vor in sehr geringer Konzentration vorkommen. Die Schätzungen liegen hier bei einer Anzahl von ungefähr 2,5 × 10⁴ Tumorzellen, die bei einem Patienten mit fortgeschrittener Krebserkrankung im Blut zirkulieren, was in etwa einer zirkulierenden Tumorzelle unter 10⁹ Blutzellen entspricht.

Die Existenz zirkulierender Tumorzellen ist bereits seit mehreren Jahrzehnten bekannt, und die Methoden zur Isolierung und Untersuchung dieser Tumorzellen wurden zunehmend verbessert. Dabei zeigte sich, dass zirkulierende Tumorzellen nicht nur als Einzelzellen auftreten können, sondern auch als Zellverbände mehrerer Tumorzellen. Diese mehrzelligen Zellverbände zirkulierender Tumorzellen werden auch als zirkulierende Tumorzellcluster (CTC-Cluster, CTCC) bezeichnet. Zirkulierende Tumorzellcluster sind zwar seltener als zirkulierende Tumoreinzelzellen, sie scheinen aber im Vergleich zu Tumoreinzelzellen mit einer bis zu 50-fach erhöhten Wahrscheinlichkeit zur Bildung von Metastasen zu führen. Die Wahrscheinlichkeit der Bildung einer Metastase scheint somit erheblich von der Zahl dieser im Blut zirkulierenden Tumorzellcluster abzuhängen. Aceto et al. konnten zeigen, dass zirkulierende Tumorzellcluster von oligoklonalen Tumorzellverbänden stammen und nicht auf intravaskulare Aggregation zurückzuführen seien (N. Aceto et al.: Circulating Tumor Cell Clusters Are Oligoclonal Precursors of Breast Cancer Metastasis; Cell 158, 1110-1122, 28.08.2014).

Die Beobachtung, dass in Blut aus der Armvene von Krebspatienten Tumorzellcluster gefunden werden konnten legte zudem nahe, dass Cluster in der Lage sein könnten kapillare Gefäße zu durchqueren. Tatsächlich konnte von Au et al. gezeigt werden, dass Cluster von zirkulierenden Tumorzellen in der Lage sind Gefäße mit kapillarähnlichen Abmessungen zu durchqueren, was bislang für unmöglich gehalten wurde (Sam H. Au et al.: "Clusters of circulating tumor cells traverse capillary-sized vessels". Proceedings of the National Academy of Sciences, 2016; 201524448 DOI: 10.1073/pnas.1524448113). Sie verwendeten hierfür eine mikrofluidische Vorrichtung mit Kanälen, die sich bis zu einer Größe von 5-10 Mikrometer verengten, was in etwa den Abmessungen der kleinsten Blutgefäße des menschlichen Körpers entspricht. Es konnte gezeigt werden, dass bei Verwendung von Tumorzellcluster, die aus Blutproben von Krebspatienten isoliert wurden, Cluster mit einer Zellanzahl von 20 Zellen oder mehr auch die kleinsten Abmessungen der Vorrichtung durchqueren konnten, ohne in den Mikrokanälen zurückgehalten zu werden. Detailliertere Untersuchungen zeigten, dass anfänglich als Zellhaufen vorliegende Tumorzellcluster in engen Kanälen eine kettenähnliche Konfiguration aneinander gereihter Zellen einnehmen können, um sich nach Durchqueren der Engstelle wieder in einen haufenähnlichen Cluster umzuordnen, der offenbar unbeschädigt zur weiteren Proliferation neigt. Die Zellen des Tumorzellclusters scheinen durch Zell-Zell-Wechselwirkungen unterschiedlicher Stärke aneinander zu haften, wobei schwächere Wechselwirkungen vorübergehend aufgegeben werden und stärkere Wechselwirkungen während des Vorliegens einer kettenähnlichen Konfiguration beibehalten werden. Die Geschwindigkeit, mit der Cluster eine Engstelle überwinden konnten hängte dabei nicht von der anfänglichen Größe des Clusters ab, sondern von der Größe der größten Zelle des Clusters, was zu dieser Vorstellung passt.

Diese Beobachtungen belegen das Potential zirkulierender Tumorzellcluster sich im Blutkreislauf zu bewegen und zur Bildung von Metastasen zu führen. Untersuchungen wie jene von Aceto et al. belegen aber auch, dass die Wahrscheinlichkeit der Metastasenbildung reduziert werden könnte, falls es gelänge zirkulierende Tumorzellcluster zu zerstören und/oder zirkulierende Tumoreinzelzellen zu inaktivieren. Unter einer "Inaktivierung" von zirkulierenden Tumorzellen oder Tumorzellcluster wird dabei verstanden, dass sie keine Fähigkeit zur Metastasenbildung mehr zeigen.

Es besteht daher das Ziel der Erfindung darin, zirkulierende Tumorzellcluster zu zerkleinern und in zirkulierende Tumoreinzelzellen umzuwandeln, sowie zirkulierende Tumorzellen zu inaktivieren, um auf diese Weise das Risiko der Metastasenbildung bei Krebserkrankungen zu verringern.

Dieses Ziel wird durch die Merkmale von Anspruch 1 erreicht. Anspruch 1 sieht eine Vorrichtung zur Zerkleinerung oder Inaktivierung von zirkulierenden Tumorzellen (CTC) oder Tumorzellclustern (CTCC) aus einem von einem Tumor betroffenen Organ oder Organteil vor, die erfindungsgemäß aus einer innerhalb eines äußeren Mantelrohres angeordneten Pumpe gebildet wird, die einen druckerhöhenden Abschnitt und eine am Pumpenausgang angeordnete druckreduzierende Drossel aufweist, sowie ausgangsseitig in ihrem durch Volumenstrom (Q) und Pumpdruck (p) gegebenen Auslegungspunkt dem Volumenstrom und dem Blutdruck des venösen Abflusses des vom Tumor betroffenen Organs oder Organteils entspricht, wobei die Pumpe als Mikroaxialpumpe mit einer entlang einer Schraubenwelle der Mikroaxialpumpe angeordneten Abfolge von druckerhöhenden Schaufeln ausgeführt ist, und die Drossel als ein radial von der Schraubenwelle der Mikroaxialpumpe abstehender Strömungswiderstand ausgeführt ist, der einen zylindrischen Abschnitt aufweist, der mit dem die Drossel umhüllenden, äußeren Mantelrohr einen kreisringzylindrischen Ringspalt ausbildet.

Die Erfindung beruht dabei auf einer Zerkleinerung der zirkulierenden Tumorzellcluster sowie Inaktivierung einzelner zirkulierender Tumorzellen durch Scherspannungen, wie sie in der erfindungsgemäßen Vorrichtung auf das beförderte Medium, im vorliegenden Fall auf das venöse Blut, ausgeübt werden. Die Pumpe fördert das venöse Blut zwar, es ist aber erfindungsgemäß nicht die Aufgabe der Pumpe den Volumenstrom oder den Pumpdruck am Pumpenausgang im Vergleich zum Pumpeneingang zu verändern, sondern Scherspannungen zu erzeugen. Scherspannungen stellen sich in jeder Flüssigkeit ein, die eine Änderung der Strömungsgeschwindigkeit erfährt. Die Scherrate stellt dabei die Differenz der Strömungsgeschwindigkeiten von zwei Flüssigkeitsschichten einer laminaren Strömung in Abhängigkeit von ihrem Abstand dar. Wenn die Flüssigkeit als ein Körper aufgefasst wird, der zwischen zwei gegeneinander verschobenen Platten angeordnet und mit ihnen durch Adhäsion verbunden ist, so wird dieser Körper bei Verschiebung der Platten eine Verformung erfahren. Die hierfür benötigte Kraft F beschleunigt die Masse des Körpers. Die Scherspannung, auch als Scherstress bezeichnet, ist die Kraft, die pro Fläche benötigt wird um diese Beschleunigung und damit Verformung zu erzeugen und damit einen bestimmten Volumenstrom in einer viskosen Flüssigkeit zu erreichen. Er ist abhängig von der Größe der verschobenen Fläche, dem Abstand der beiden Flächen und der dynamischen Viskosität der dazwischen befindlichen Flüssigkeit. Typische Werte für Scherspannungen im venösen Blutkreislauf liegen bei 0.1-0.25 Pa.

Erste experimentelle Untersuchungen der Erfinder haben gezeigt, dass durch Scherspannungen im Bereich von 200-500 Pa, die in mikrofluidischen Vorrichtungen nachgebildet werden können, Tumorzellcluster, die aus Blutproben von Krebspatienten isoliert wurden, zerstört und in Tumoreinzelzellen umgewandelt werden konnten. Zudem konnte sogar die Zerstörung und somit Inaktivierung zirkulierender Tumoreinzelzellen beobachtet werden.

Zu hohe Scherspannungen könnten aber auch hämolytisch wirken und somit Blutbestandteile schädigen. Bekannt ist beispielsweise, dass der auf Erythrozyten ausgeübte Scherstress indirekt die Aggregation von Blutplättchen beeinflussen kann, die ihrerseits ebenfalls scherstressabhängig ist. Scherstress verursacht auch eine spindelförmige Verformung der Erythrozyten.

Die Scherspannungen, die im Experiment eine Zerkleinerung von Tumorzellcluster sowie eine Inaktivierung einzelner Tumorzellen herbeiführen konnten, scheinen jedoch ausreichend niedrig zu sein, um die sonstigen Blutbestandteile nicht oder kaum dauerhaft zu schädigen. Es wird vermutet, dass im Gegensatz zu Erythrozyten, Leukozyten, Thrombozyten oder Endothelzellen, die an die Hämodynamik adaptiert sind, die mechanischen Eigenschaften von zirkulierenden Tumorzellen und Tumorzellclustern nicht perfekt an die Strömung im vaskulären System angepasst sind und insbesondere eine begrenzte Widerstandsfähigkeit gegenüber Scherspannungen aufweisen. Dieser mechanische Unterschied wird erfindungsgemäß ausgenutzt. In bislang unveröffentlichten Vorversuchen mit kultivierten Tumorzellen bzw. Tumorzellclustern konnten die Erfinder einerseits zeigen, dass durch zeitlich begrenzte Einwirkung von hohem Flüssigkeitsscherstress auf Tumorzellcluster diese zerstört werden können, so dass nur mehr Einzelzellen vorliegen. Bei noch höherem Scherstress kommt es sogar zur Zerstörung der zirkulierenden Tumoreinzelzellen. Bei diesen Experimenten zeigte sich andererseits auch, dass die Zerstörung der Tumorzellcluster bzw. der zirkulierenden Tumorzellen unmittelbar mit zwei Parametern korrelierte: Zum Einen die Höhe des Flüssigkeitsscherstresses (der Schubspannung), und zum Anderen die Dauer der Einwirkung des Scherstresses. Der genaue Aufbau und Ergebnisse der Versuche wird in weiterer Folge noch genauer erläutert werden. Unter den gleichen Bedingungen wurde humanes Blut von freiwilligen Spendern ebenfalls Scherstress ausgesetzt. Danach wurde das Blut untersucht. Es wurden sowohl Erythrozyten, Leukozyten, Thrombozyten und deren Abbauprodukte im Serum quantifiziert. Dabei konnte keine Hämolyse festgestellt werden. Selbst bei Bedingungen bei welchen 90% der zirkulierenden Tumorzellen zerstört wurden war kein Unterschied zu ungeschertem Blut feststellbar. Es konnte somit gezeigt werden, dass ein therapeutisches Fenster für den Scherstress existiert, in welchem zirkulierende Tumorzellen und Tumorzellcluster, nicht aber das Blut geschädigt werden.

Erfindungsgemäß wird dieser Effekt ausgenutzt, indem in der Pumpe Scherspannungen im Bereich von 200-500 Pa nachgebildet werden, um Tumorzellcluster zu zerstören und in Tumoreinzelzellen umzuwandeln. Die Erfindung sieht nun vor eine solche Pumpe in der Nähe des Tumors zu platzieren. Vorzugsweise wird die Pumpe begleitend bei einer Operation zur Entfernung eines Primärtumors eingesetzt, um bei der Operation in den Blutkreislauf freigesetzte Tumorzellcluster unmittelbar zu zerkleinern und so das häufig nach Operationen erhöht auftretende Risiko der Metastasenbildung gezielt zu reduzieren. Die Pumpe kann in weiterer Folge beim Abschluss der Operation zur chirurgischen Entfernung des Primärtumors wieder entfernt werden, oder über einen geeigneten Zeitraum noch im venösen Abfluss belassen werden, um sie erst später zu entfernen.

Da der Eintritt allfällig freigesetzter Tumorzellcluster in den Blutkreislauf des Patienten über den venösen Abfluss des vom Tumor betroffenen Organs oder Organteiles erfolgt, wird die Pumpe erfindungsgemäß im venösen Abfluss des betroffenen Organs oder Organteiles platziert. Es handelt sich dabei somit um den Niederdruckbereich des Blutkreislaufs. In bekannter Weise sind die Druckverhältnisse im Blutkreislauf sehr unterschiedlich. Das Herz funktioniert dabei in erster Linie als Presspumpe und weniger als Saugpumpe, wobei es in jeder Auswurfphase beim herzgesunden Erwachsenen durchschnittlich etwa 70 Milliliter Blut aus der linken Herzkammer in die Aorta presst. Diese Blutmenge übt Druck auf die Blutsäule in der Aorta beziehungsweise in den von ihr abgehenden Arterien aus. Damit wird das Blut weiter durch das Gefäßsystem über den arteriellen Schenkel des Kapillarsystems in die Venolen und von dort über die Venen in Richtung des rechten Herzvorhofes "geschoben". Die Venen führen das Blut von allen Organen und entfernten Körperstellen zurück zum Herzen. Je größer der Venendurchmesser in Richtung Herzen wird, desto geringer ist der dort herrschende Druck. Bei einem gesunden, liegenden Probanden beträgt der Druck im kapillaren Venenteil etwa 20 mmHg, sinkt in der Leistengegend auf etwa 8-12 mmHg ab, beträgt im Bauchraum (intraabdominal) noch etwa 3-5 mm Hg und im rechten Herzvorhof nur noch 2 mmHg.

Grundsätzlich wird angestrebt die erfindungsgemäße Vorrichtung möglichst nahe des vom Tumor betroffenen Organs oder Organteiles zu platzieren. Freilich muss die entsprechende Vene für die entsprechende Platzierung der Pumpe hinsichtlich Größe und Zugänglichkeit geeignet sein, zudem können auch physiologische Gegebenheiten oder medizinische Gründe für einen bestimmten Ort der Platzierung der erfindungsgemäßen Pumpe im venösen Abfluss eines Organs oder Organteiles sprechen. Als besonders geeignet erscheinen dabei die Leberpfortader (V. portae hepatis), die Nierenvene (V. renalis), die untere Hohlvene (V. cava inferior) und die obere Hohlvene (V. cava superior). Bei der oberen Hohlvene erscheint insbesondere eine Platzierung der erfindungsgemäßen Pumpe im Bereich des Venenwinkels (Angulus venosus) als besonders vorteilhaft, da im Venenwinkel die großen Lymphsammelstämme münden und somit auch die Wahrscheinlichkeit lymphogener Metastasierung verringert werden kann. Es ist aber auch denkbar mehrere Vorrichtungen gemäß der Erfindung an unterschiedlichen Stellen im venösen Abfluss eines vom Tumor betroffenen Organs oder Organteiles zu platzieren.

Da im Gegensatz zu den Arterien der Blutdruck in den Venen sehr niedrig ist, besitzen die Venen eine wesentlich dünnere Wand als Arterien, zudem fließt das Blut in den Venen durch den niedrigen Blutdruck nur sehr langsam. Daher wird erfindungsgemäß eine Pumpe vorgeschlagen, die in ihrem durch Volumenstrom und Pumpdruck gegebenen Auslegungspunkt dem Volumenstrom und dem Blutdruck des venösen Abflusses des vom Tumor betroffenen Organs entspricht. Bekanntlich verfügt jede Pumpe über einen Auslegungspunkt, der in der Regel über den geforderten Volumenstrom und dem gewünschten Pumpdruck definiert ist. Anhand des so vorgegebenen Auslegungspunktes und dem zu fördernden Medium - im gegenständlichen Fall Blut - können in weiterer Folge Pumpenvariablen wie beispielsweise der Durchmesser und die Länge der Pumpe, die Auslegung des Rotors und die Drehzahl der Pumpe festgelegt werden und für die betreffende Pumpe die Pumpenkennlinie, die den Verlauf des Volumenstroms in Abhängigkeit vom Förderdruck abseits des Auslegungspunktes angibt, ermittelt werden.

Im praktischen Einsatz wird die Pumpe schließlich an einem vorgegebenen Betriebspunkt betrieben, der in der Regel dem Auslegungspunkt entspricht. Erfindungsgemäß wird die Pumpe so betrieben, dass sie ausgangsseitig in ihrem durch Volumenstrom Q und Pumpdruck p gegebenen Betriebspunkt dem Volumenstrom und dem Blutdruck des venösen Abflusses des vom Tumor betroffenen Organs oder Organteils entspricht. Nach Einführung der erfindungsgemäßen Pumpe in den venösen Abfluss des vom Tumor betroffenen Organs oder Organteiles wird das Blut am Pumpeneingang abgenommen und am Pumpenausgang wieder ausgeworfen, aber ohne den Volumenstrom oder den Pumpdruck am Pumpenausgang im Vergleich zum Pumpeneingang nennenswert zu verändern, sondern um Scherkräfte zu erzeugen, die zwar die zirkulierenden Tumorzellcluster zerstören, die sonstigen Blutbestandteile aber nicht beschädigen. Zusätzlich ist dafür zu sorgen, dass die Durchblutung des Körpers des Patienten nicht negativ beeinträchtigt wird. Stauungen und lokale oder gar systemische Hypertonien sind unter allen Umständen zu vermeiden. Dazu muss sich das Gerät der lokalen Kreislaufregelung im Körper anpassen. Daher wird vorzugsweise vorgeschlagen, dass vor der Pumpe und nach der Drossel jeweils ein Drucksensor vorgesehen ist, sowie eine Regeleinheit, die die Druckdifferenz der von den beiden Drucksensoren gemessenen Druckwerte durch Variation der Drehzahl der Schraubenwelle auf einen vorgegebenen Sollwert regelt. Der für die Druckdifferenz vorgegebene Sollwert beträgt dabei vorzugsweise 0. So erscheint die erfindungsgemäße Vorrichtung für die natürliche Durchblutung als nicht existent. Die Funktion ist voraussichtlich über einen weiten Regelbereich gegeben. Das liegt, wie in den oben genannten Vorversuchen festgestellt wurde, daran, dass die Zerstörungsleistung der erfindungsgemäßen Vorrichtung vom Scherstress und von der Verweildauer auf der rotierenden Drossel abhängig ist. Bei erhöhtem Blutstrom muss, damit der Differenzdruck bei 0 gehalten wird, die Drehzahl hochgeregelt werden. Dadurch wird mehr durch die Pumpe gefördert und so verringert sich die Verweildauer auf der Drossel. Gleichzeitig steigt aber der Scherstress. Erfindungsgemäß wird dies sichergestellt, indem die Pumpe als Mikroaxialpumpe mit einer entlang einer Schraubenwelle der Mikroaxialpumpe angeordneten Abfolge von druckerhöhenden Schaufeln ausgeführt ist, und die Drossel als ein radial von der Schraubenwelle der Mikroaxialpumpe abstehender Strömungswiderstand. Die Drossel und die Mikroaxialpumpe sind somit über die gemeinsame Schraubenwelle hinsichtlich ihrer Drehzahl zwangsgeführt, das heißt, dass sich die Drossel und die Mikroaxialpumpe stets mit derselben Drehzahl drehen. Steigt der Volumenstrom aufgrund höherer Drehzahl der Mikroaxialpumpe, sinkt zwar die Verweildauer, aber die Scherspannung insbesondere im Ringspalt zwischen der Drossel und dem äußeren Mantelrohr steigt aufgrund der ebenfalls höheren Drehzahl der Drossel. Sinkt hingegen der Volumenstrom aufgrund niedrigerer Drehzahl der Mikroaxialpumpe, erhöht sich zwar die Verweildauer, aber die Scherspannung insbesondere im Ringspalt zwischen der Drossel und dem äußeren Mantelrohr sinkt aufgrund der ebenfalls niedrigeren Drehzahl der Drossel. Solange die Pumpenkennlinie mit hinreichender Genauigkeit linearisiert werden kann, bleibt die Zerstörungswirkung jedoch annähernd konstant. In den oben genannten Vorversuchen wurde eine rotierende Drossel mit Drehzahlregelung und eine davon getrennte, unabhängig zu regelnde Pumpe benutzt. So konnten Scherstress und Verweildauer unabhängig eingestellt werden. Wesentlich für die Auslegung der erfindungsgemäßen Vorrichtung ist auch, dass das Blut immer unter Druck gehalten wird, da Unterdruck zu Hämolyse führt. Außerdem soll im Bereich der Axialpumpe möglichst konstanter Scherstress herrschen.

Da Kavitation unbedingt zu vermeiden ist wird vorzugsweise vorgeschlagen, dass innerhalb des äußeren Mantelrohres ein koaxiales, inneres Mantelrohr vorgesehen ist, das an den Schaufeln der Mikroaxialpumpe befestigt ist. Diese Konstruktion hat den Vorteil einer sehr gleichmäßigen Verteilung des Scherstress und verhindert Kavitation an den Außenkanten der Schraube. Die Schraubenwelle erstreckt sich in axialer Richtung über die axiale Erstreckung des inneren Mantelrohres hinaus und geht in diesem Bereich in die druckreduzierende Drossel über, die einen zylindrischen Abschnitt aufweist, der mit dem die Drossel umhüllenden, äußeren Mantelrohr einen kreisringzylindrischen Ringspalt ausbildet.

Da die erfindungsgemäße Pumpe in den venösen Abfluss eines Organs oder Organteiles intrakorporal zu platzieren ist, werden entsprechend kleine Abmessungen erforderlich, wobei entsprechende Pumpen als Mikropumpen bezeichnet werden. Bauliche Ausführungen solcher Mikropumpen sind an sich für den Bereich der Herzunterstützungssysteme bekannt, bei denen implantierte Pumpen die Herztätigkeit unterstützen oder sogar ersetzen sollen. Zumeist handelt es sich dabei um Linksherzunterstützungssysteme (engl. Left Ventricle Assist Devices, LVAD), bei denen eine Pumpe Blut aus der linken Herzkammer (dem linken Ventrikel) in die Aorta pumpt. Der Auslegungs- und folglich der Betriebspunkt der hier geforderten Pumpen unterscheidet sich aber grundsätzlich von jenem der erfindungsgemäß vorgesehenen Pumpe, da sie bei bekannten Herzunterstützungssystemen im Hochdruckbereich des Blutkreislaufs angeordnet werden und für einen höheren Volumenstrom und einen höheren Pumdruck ausgelegt werden. Weitere Pumpen wurden in der WO 95/29716 A1 und der US 2016/331378 A1 beschrieben.

Im Rahmen der vorliegenden Erfindung beträgt der Volumenstrom Q der Pumpe vorzugsweise 0.1-1.5 Liter/Minute und der Pumpdruck p 4-20 hPa. Diese Strömungsverhältnisse treten im venösen Abfluss der meisten Organe des Menschen auf, so beträgt etwa der Volumenstrom in der Leberpfortader eines erwachsenen Menschen etwa 0.8 l/min bei einem Blutdruck von etwa 4-8 hPa, im venösen Abfluss der Leber etwa 1.3 l/min und im venösen Abfluss der Niere etwa 0.5 l/min. Im venösen Blutkreislauf kann der Volumenstrom aber auch deutlich abweichende Werte aufweisen, so beträgt der Volumenstrom in der oberen Hohlvene etwa 2 l/min, in der unteren Hohlvene etwa 3 l/min und im Lungenkreislauf bis zu 5l/min. Der genaue Wert des Auslegungspunktes der erfindungsgemäßen Pumpe hängt somit vom Volumenstrom und dem Druck am Ort der Platzierung der Pumpe im venösen Abfluss des vom Tumor betroffenen Organs oder Organteiles ab, wie noch näher ausgeführt werden wird.

Da Volumenstrom und Blutdruck aber nicht nur innerhalb des venösen Blutkreislaufes desselben Menschen, sondern auch von Mensch zu Mensch je nach Alter, Gewicht oder körperlicher Verfassung stark variieren, ist es denkbar den genauen Wert des Auslegungspunktes der erfindungsgemäßen Pumpe an einen bestimmten Patienten individuell anzupassen. So ist zum einen das exakte Kaliber des betroffenen Gefäßes, als auch die Konstitution des Patienten und das individuelle Herzminutenvolumen zu berücksichtigen. Neuere diagnostische Verfahren ermöglichen die lokale Bestimmung von Gefäßdurchmesser, Blutdruck und Volumenstrom an beliebigen Stellen des venösen Blutkreislaufes, sodass punktgenau lokale Strömungsbedingungen ermittelt werden können. Es ist somit denkbar diese Messwerte für die Bestimmung des Auslegungspunktes der erfindungsgemäßen Pumpe heranzuziehen und die Pumpe in weiterer Folge anhand der bekannten Scherspannungen, die in der Pumpe zur Zerkleinerung und Inaktivierung der Tumorzellen und Tumorzellcluster sichergestellt werden müssen, zu entwerfen. Um die erfindungsgemäße Vorrichtung individualisiert herstellen zu können, können etwa additive und subtraktive Fertigungsverfahren verwendet werden, beispielsweise in Form von 3D-Metalldruck, sodass eine Fertigung der kritischen und individuell zu dimensionierenden Bestandteile der erfindungsgemäßen Vorrichtung innerhalb kürzester Zeit möglich ist.

Die Erfindung wird in weiterer Folge anhand von Ausführungsbeispielen mithilfe der beiliegenden Zeichnungen näher erläutert. Hierbei zeigen die
Fig. 1 eine schematische Darstellung einer Ausführungsform einer Anordnung zur Anwendung der erfindungsgemäßen Vorrichtung,
Fig. 2 eine Darstellung einer ersten Ausführungsform einer erfindungsgemäßen Mikroaxialpumpe mit Drossel,
Fig. 3a eine vergrößerte Darstellung einer Ausführungsform einer erfindungsgemäßen Mikroaxialpumpe mit Drossel gemäß der Fig. 2,
Fig. 3b eine Darstellung einer zweiten Ausführungsform einer erfindungsgemäßen Mikroaxialpumpe mit Drossel,
Fig. 4A eine mikroskopische Aufnahme eines Tumorzellclusters,
Fig. 4B eine mikroskopische Aufnahme des in Tumoreinzelzellen umgewandelten Tumorzellclusters der Fig. 4A nach Passieren einer mikrofluidischen Vorrichtung bei Scherraten wie sie auch bei der erfindungsgemäßen Vorrichtung auftreten,
Fig. 5 eine Darstellung zum grundsätzlichen Funktionsprinzip der erfindungsgemäßen Vorrichtung,
Fig. 6 eine Darstellung zur Erläuterung der Platzierung der erfindungsgemäßen Vorrichtung am Beispiel des Verdauungssystems,
Fig. 7 eine weitere Darstellung zur Erläuterung der Platzierung der erfindungsgemäßen Vorrichtung im Blutkreislauf,
Fig. 8A eine mikroskopische Aufnahme kultivierter Tumorzellen (LM1-Zellen aus einem Colonkarzinom), die in der Kultur deutlich erkennbare Tumorzellcluster bilden,
Fig. 8B eine mikroskopische Aufnahme kultivierter Tumorzellen nach kurzer Applikation von Scherstress, wobei praktisch nur mehr Einzelzellen vorliegen,
Fig. 8C eine mikroskopische Aufnahme kultivierter Tumorzellen nach Applikation von einem gegenüber Fig. 8B höherem Scherstress, wobei Zellschädigungen und somit die Inaktivierung einzelner Tumorzellen zu erkennen sind, und die
Fig. 8D eine Zusammenfassung unterschiedlicher Experimente, wobei insbesondere der prozentuelle Anteil aller mikroskopisch sichtbaren Partikel nach der Zahl der Zellen, aus welchen sie bestehen, gezeigt wird.

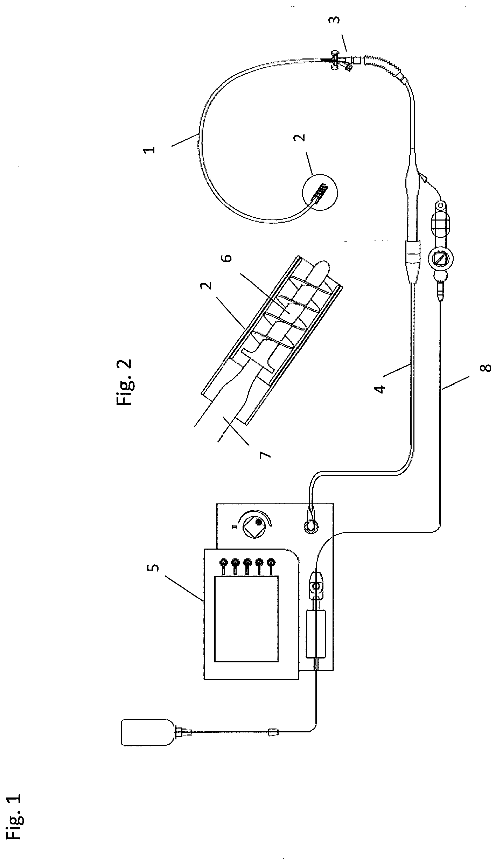

Zunächst wird auf die Fig. 1 Bezug genommen, die eine schematische Darstellung einer Ausführungsform einer möglichen Anordnung zur Anwendung der erfindungsgemäßen Vorrichtung zeigt. Auf der rechten Seite der Fig. 1 ist der flexible Venenkatheter 1 zu sehen, an dessen freiem Ende die erfindungsgemäße Pumpe 2 angeordnet ist. Die Pumpe 2 ist als Mikroaxialpumpe ausgeführt und in der Fig. 1 mit einem Kreis markiert und in der Fig. 2 vergrößert dargestellt. Der Luer-Lock-Anschluss 3 für den Venenkatheter befindet sich außerhalb des Patienten. Am Luer-Lock-Anschluss 3 wird die Verbindungsleitung 4 zur Steuer- und Überwachungseinheit 5 angeschlossen. Über den Venenkatheter 1 und die Verbindungsleitung 4 wird eine elektrische Verbindung 7 zwischen der Steuer- und Überwachungseinheit 5 und der Pumpe 2 gelegt (siehe auch Fig. 2). Über die Steuer- und Überwachungseinheit 5 lassen sich auf diese Weise Betriebsparameter wie die Drehzahl der Schraubenwelle 6 der als Mikroaxialpumpe ausgeführten Pumpe 2 steuern. Es wäre aber auch denkbar lokale Venendruckmessungen über den Venenkatheter durchzuführen und die Messwerte über die Steuer- und Überwachungseinheit 5 darzustellen. Optional kann ein Zuspritzsystem 8 für die Zufuhr von Medikamenten wie etwa Chemotherapeutika oder Blutverdünnern, oder auch für die Zufuhr von Glukoselösungen und dergleichen vorgesehen sein.

Alternativ könnte die erfindungsgemäße Pumpe 2 auch implantiert werden, indem die Pumpe 2 chirurgisch in die Vene eingesetzt wird, oder die Vene am Ort der Pumpe 2 durchtrennt und an beide Seiten der Pumpe 2 befestigt wird.

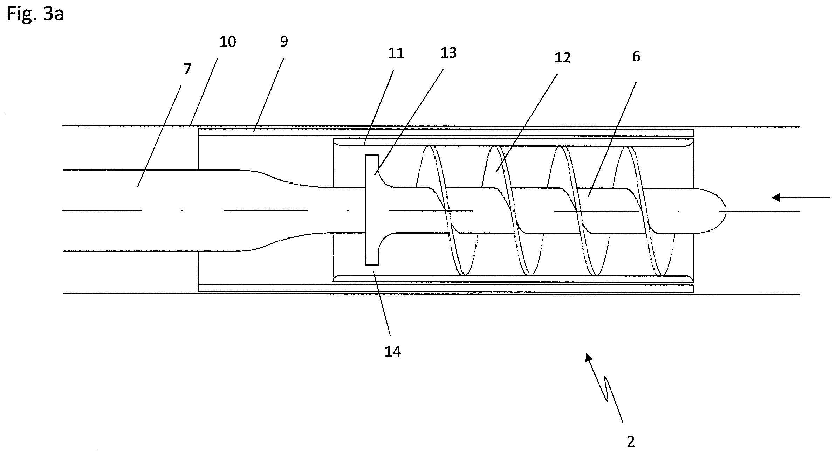

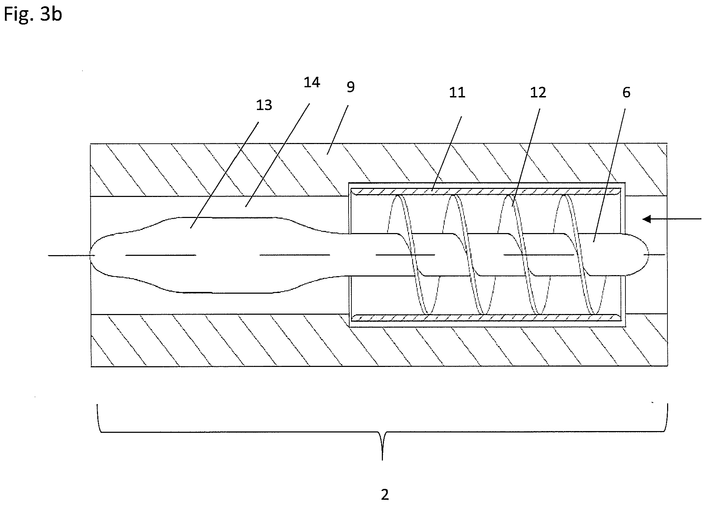

Anhand der Fig. 3a und 3b werden mögliche Ausführungsformen der als Mikroaxialpumpe ausgeführten Pumpe 2 erläutert. Gemäß der gezeigten Ausführungsformen ist ein äußeres Mantelrohr 9 vorgesehen, das am vorgesehenen Ort der Platzierung der Mikroaxialpumpe stationär innerhalb der Venenwand 10 angeordnet wird. Ein inneres Mantelrohr 11 ist an der Schraubenwelle 6 und/oder den Schaufeln 12 der Mikroaxialpumpe befestigt und dreht sich mit der Schraubenwelle 6 mit. Die Schraubenwelle 6 wird über einen (in der Fig. 3a und 3b nicht dargestellten) Antrieb in Rotation versetzt, der über die elektrische Verbindung 7 mit Strom versorgt wird. Der Eingang der Pumpe 2 befindet sich in Bezug auf die Fig. 3a und 3b auf der rechten Seite, wobei die Fließrichtung des venösen Blutes mit einem Pfeil angezeigt ist. Der Ausgang der Pumpe 2 befindet sich in Bezug auf die Fig. 3a und 3b auf der linken Seite.

Da die Venenwand 10 im venösen Abfluss eines Organs oder Organteiles mitunter sehr dünn sein kann und im Zuge des Einsatzes der erfindungsgemäßen Pumpe 2 freilich nicht beschädigt werden soll, ist am Ausgang der Pumpe 2 eine druckreduzierende Drossel 13 angeordnet. Diese Maßnahme ermöglicht es auch den Pumpdruck p zur Erhöhung der Scherkräfte innerhalb der Mikroaxialpumpe 2 gezielt zu erhöhen, aber dennoch moderate und auf den venösen Abfluss VA abgestimmte Druckverhältnisse am Pumpausgang sicher zu stellen. Die Drossel 13 ist als ein radial von der Schraubenwelle 6 der Pumpe 2 abstehender Strömungswiderstand ausgeführt, der einen zylindrischen Abschnitt aufweist, der mit dem die Drossel 13 umhüllenden, äußeren Mantelrohr 9 einen kreisringzylindrischen Ringspalt 14 ausbildet. In diesem Ringspalt 14 herrscht entsprechender Scherstress.

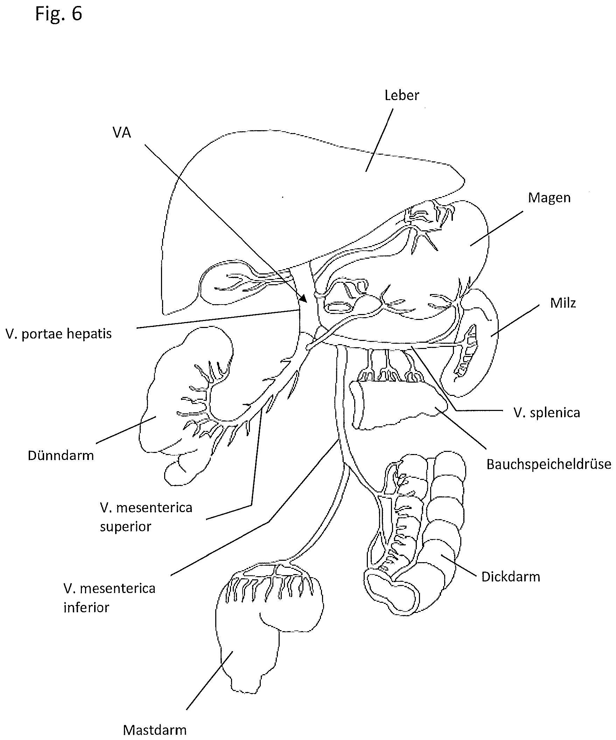

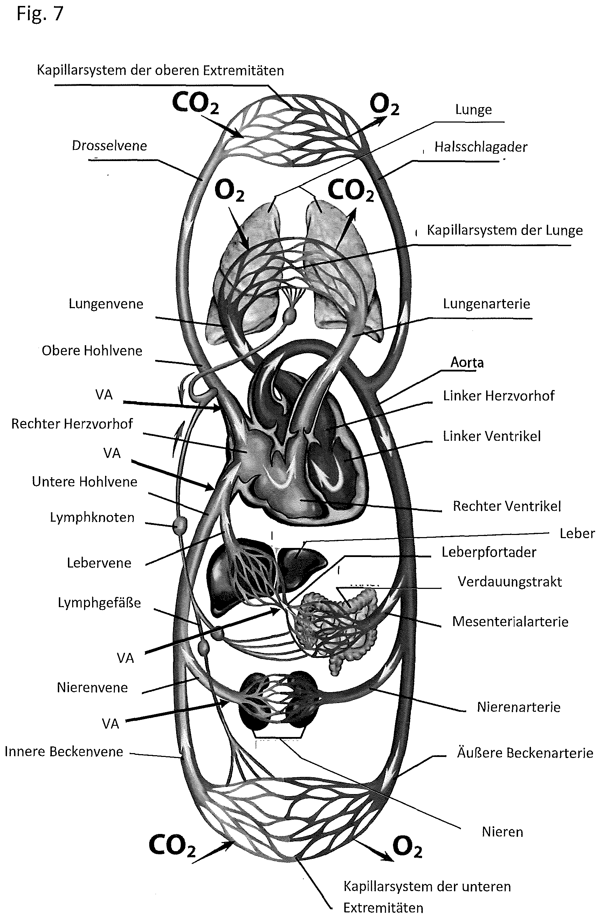

Die erfindungsgemäße Pumpe 2 entspricht in ihrem durch Volumenstrom Q und Pumpdruck p gegebenen Auslegungspunkt dem Volumenstrom und dem Blutdruck des venösen Abflusses VA des vom Tumor betroffenen Organs (siehe auch Fig. 6 und 7). Im Rahmen der vorliegenden Erfindung beträgt der Volumenstrom Q der Pumpe 2 vorzugsweise 0.1-1.5 Liter/Minute und der Pumpdruck p 4-20 hPa. Der genaue Wert des Auslegungspunktes hängt vom Volumenstrom und dem Druck am Ort der Platzierung der Pumpe 2 im venösen Abfluss VA des vom Tumor betroffenen Organs oder Organteiles ab. Die Drossel 13 wird bei der Wahl des Auslegungspunktes der Pumpe 2 freilich berücksichtigt, sodass sich die genannten Werte für den Volumenstrom Q der Pumpe 2 von 0.1-1.5 Liter/Minute und den Pumpdruck p von 4-20 hPa auf die Verhältnisse in Strömungsrichtung gesehen nach der Drossel 13 beziehen.

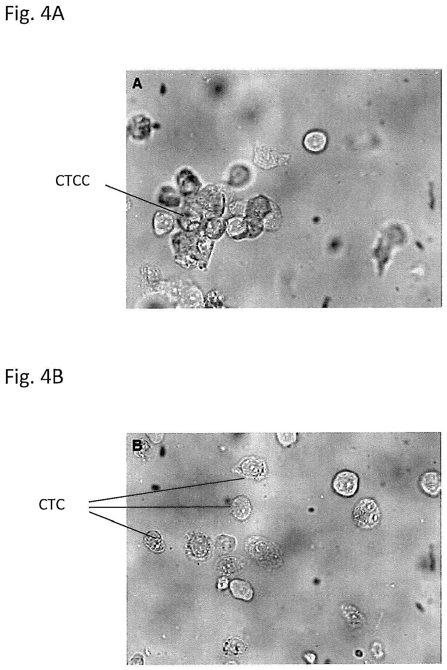

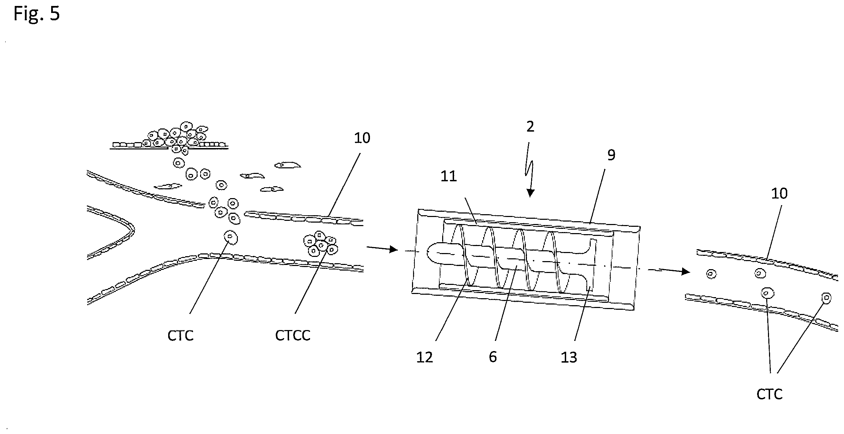

Das grundlegende Funktionsprinzip der erfindungsgemäßen Vorrichtung wird anhand der Fig. 4 und 5 erläutert. Die Fig. 4 zeigt experimentelle Ergebnisse zur Zerkleinerung von zirkulierenden Tumorzellcluster CTCC durch Scherspannungen, wobei die Fig. 4A eine mikroskopische Aufnahme eines aus Blutproben von Krebspatienten isolierten Tumorzellclusters CTCC vor Passieren einer mikrofluidischen Vorrichtung bei Scherraten, wie sie durch die erfindungsgemäße Vorrichtung nachgebildet werden, und die Fig. 4B eine mikroskopische Aufnahme des in Tumoreinzelzellen CTC umgewandelten Tumorzellclusters CTCC der Fig. 4A nach Passieren dieser mikrofluidischen Vorrichtung. Der Vergleich der Fig. 4A mit der Fig. 4B zeigt, dass Tumorzellcluster CTCC zerstört und in Tumoreinzelzellen CTC umgewandelt werden konnten. Dabei wird angemerkt, dass sich die in der Fig. 4B berührenden Tumoreinzelzellen CTC unabhängig voneinander bewegen, sobald die Kulturschale bewegt wird, also nicht aneinander haften. Bei einem Beobachtungszeitraum von 60 Minuten konnte dabei keine neuerliche Aggregation der Tumoreinzelzellen CTC zu Tumorzellcluster CTCC beobachtet werden. Andererseits sind die Scherspannungen, die im Experiment eine Zerkleinerung von Tumorzellcluster CTCC herbeiführen konnten, auch niedrig genug um die sonstigen Blutbestandteile nicht zu schädigen. Dieses therapeutische Fenster wird durch die erfindungsgemäße Vorrichtung ausgenutzt.

Eine modellhafte Illustration dieses Vorganges ist in der Fig. 5 dargestellt. Auf der linken Seite der Fig. 5 wird der Vorgang gezeigt, bei dem etwa im Zuge der operativen Entfernung eines Primärtumors Tumorgewebe sowie der im Tumorgewebe und dem umgebenden Gewebe vorhandenen Blutgefäße verletzt werden, was mit einer entsprechenden Freisetzung von zirkulierenden Tumorzellen CTC in den Blutkreislauf und einer damit verbundenen Erhöhung des Risikos der Metastasenbildung verbunden sein kann. Die zirkulierenden Tumorzellen CTC können dabei auch als zirkulierende Tumorzellcluster CTCC freigesetzt werden und sich in dieser Konfiguration durch den Blutkreislauf bewegen. Erfindungsgemäß ist jedoch eine als Mikroaxialpumpe ausgeführte Pumpe 2 im venösen Abfluss VA des vom Tumor betroffenen Organs angeordnet (siehe auch Fig. 6 und 7), die Scherspannungen auf die im Blut enthaltenen Inhaltsstoffe ausüben, die zwar die sonstigen Blutbestandteile nicht schädigen, auf die zirkulierenden Tumorzellen CTC und Tumorzellcluster CTCC jedoch eine zersetzende und somit inaktivierende Wirkung ausüben. Nach dem Passieren der Pumpe 2 finden sich somit keine zirkulierenden Tumorzellcluster CTCC mehr in der Blutbahn, sondern weitestgehend inaktivierte zirkulierende Tumoreinzelzellen CTC mit bedeutend geringerem Risiko der Metastasenbildung.

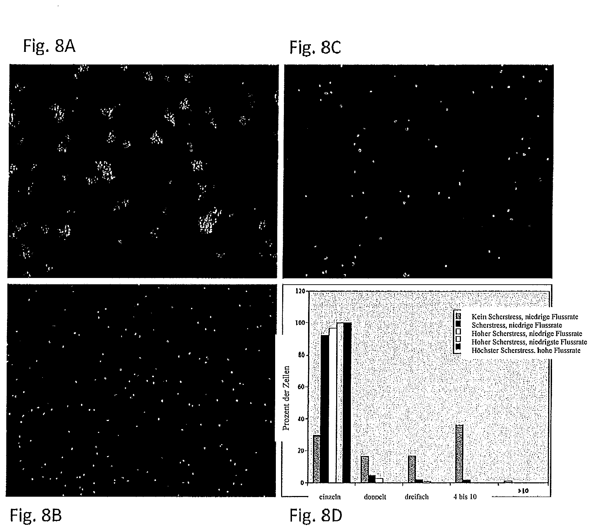

Die Fig. 8A zeigt hierzu eine mikroskopische Aufnahme kultivierter Tumorzellen (LM1-Zellen aus einem Colonkarzinom), die in der Kultur deutlich erkennbare Tumorzellcluster bilden. Die Fig. 8B zeigt eine mikroskopische Aufnahme kultivierter Tumorzellen nach kurzer Applikation von Scherstress, wobei praktisch nur mehr Einzelzellen vorliegen, die Fig. 8C eine mikroskopische Aufnahme kultivierter Tumorzellen nach Applikation von einem gegenüber Fig. 8B höherem Scherstress, wobei Zellschädigungen und somit die Inaktivierung einzelner Tumorzellen zu erkennen sind, und die Fig. 8D eine Zusammenfassung unterschiedlicher Versuche, wobei insbesondere der prozentuelle Anteil aller mikroskopisch sichtbaren Partikel nach der Zahl der Zellen, aus welchen sie bestehen, gezeigt wird. Diese Versuche zeigen, dass durch zeitlich begrenzte Einwirkung von hohem Flüssigkeitsscherstress auf Tumorzellcluster CTCC diese zerstört werden können, so dass nur mehr Tumoreinzelzellen CTCs vorliegen. Bei noch höherem Scherstress kommt es sogar zur Zerstörung der Tumoreinzelzellen CTCs.

Bei diesen Versuchen zeigte sich ferner, dass die Zerstörung der Tumorzellcluster CTCC bzw. der Tumoreinzelzellen CTCs unmittelbar mit zwei Parametern korrelierte: Zum Einen die Höhe des Flüssigkeitsscherstresses (der Schubspannung), und zum Anderen die Dauer der Einwirkung des Scherstresses. Höhere Scherspannung bei kürzerer Verweildauer in der Pumpe 2 führten dabei zur selben inaktivierenden Wirkung der Pumpe 2 wie niedrigere Scherspannung bei längerer Verweildauer in der Pumpe 2. Das ermöglicht eine Regelung der Pumpe 2 innerhalb eines breiten therapeutischen Bereiches, indem der Druck eingangsseitig und ausgangsseitig der Pumpe 2 gemessen und der Differenzdruck mithilfe einer Regeleinheit bei 0 gehalten wird. Bei erhöhtem Blutstrom wird die Drehzahl hochgeregelt. Dadurch wird mehr durch die Pumpe 2 gefördert und so verringert sich die Verweildauer auf der Drossel 13. Gleichzeitig steigt aber der Scherstress. Solange die Pumpenkennlinie mit hinreichender Genauigkeit linearisiert werden kann, bleibt die Zerstörungswirkung der erfindungsgemäßen Vorrichtung annähernd konstant. Die Regeleinheit kann dabei autonom arbeiten und gewährleistet somit, dass sich die erfindungsgemäße Vorrichtung der lokalen Kreislaufregelung im Körper anpassen kann. Insbesondere wird eine negative Beeinträchtigung der Durchblutung des Körpers des Patienten sowie Stauungen und lokale oder gar systemische Hypertentionen vermieden. Vielmehr erscheint die erfindungsgemäße Vorrichtung für die natürliche Durchblutung als nicht existent.

Wie bereits erwähnt wurde, wird im Rahmen der Erfindung grundsätzlich angestrebt die erfindungsgemäße Vorrichtung möglichst nahe des vom Tumor betroffenen Organs oder Organteiles zu platzieren. Freilich muss die entsprechende Vene für die entsprechende Platzierung der Pumpe 2 hinsichtlich Größe und Zugänglichkeit geeignet sein, zudem können auch physiologische Gegebenheiten oder medizinische Gründe für einen bestimmten Ort der Platzierung der erfindungsgemäßen Pumpe 2 im venösen Abfluss VA eines Organs oder Organteiles sprechen, wie anhand der Fig. 6 und Fig. 7 erläutert werden soll. Als besonders geeignet erscheint dabei etwa die Leberpfortader (*V. portae hepatis*), wie in dem mit Pfeil angezeigten Bereich der Fig. 6 gezeigt wird. Die Leberpfortader sammelt das venöse Blut aus den unpaaren Bauchorganen wie dem Magen, dem Dünndarm über die obere Mesenterialvene (*V. mesenterica superior*), dem Dickdarm und Teilen des Mastdarms über die untere Mesenterialvene (*V. mesenterica inferior*), der Bauchspeicheldrüse und der Milz über die Milzvene (*V. splenica*) und führt es der Leber zu. Sofern die physiologischen Voraussetzungen hierfür bestehen kann die erfindungsgemäße Pumpe 2 in einer oder mehreren der genannten venösen Abflüsse VA angeordnet werden, die Platzierung in der Leberpfortader vermindert jedoch zuverlässig die hämatogene Metastasierung aufgrund von zirkulierenden Tumorzellclustern CTCC in der Leber.

Des Weiteren erscheinen die Nierenvene (*V. renalis*), die untere Hohlvene (*V. cava inferior*) und die obere Hohlvene (*V. cava superior*) als besonders geeignet für die Platzierung der erfindungsgemäßen Pumpe 2, wie in den mit Pfeilen angezeigten Bereichen der Fig. 7 gezeigt wird. Bei der oberen Hohlvene erscheint insbesondere eine Platzierung der erfindungsgemäßen Pumpe 2 im Bereich des Venenwinkels (*Angulus venosus*) als besonders vorteilhaft. Als Venenwinkel werden beim Menschen die beiden Stellen (rechte/linke Körperhälfte) im Blutgefäßsystem bezeichnet, wo sich jeweils die Drosselvene (*Vena jugularis interna*) mit der Schlüsselbeinvene (*Vena subclavia*) zur Vena brachiocephalica vereinigt. Hier münden auch die großen Lymphsammelstämme. Der linke Venenwinkel ist insofern der bedeutsamere, da hier (neben anderen) der Lymphsammelstamm *Ductus thoracicus* einmündet, der Lymphe aus der gesamten unteren Körperhälfte transportiert. In den rechten Venenwinkel mündet dagegen nur ein kleineres Lymphgefäß, das Lymphe aus dem rechten Arm, der rechten Thoraxseite und der rechten Halsseite (*Ductus lymphaticus dexter*) führt, ein. Im weiteren Verlauf vereinigen sich rechte und linke Vena brachiocephalica zur oberen Hohlvene, die in den rechten Herzvorhof mündet (siehe Fig. 7). Da im Venenwinkel die großen Lymphsammelstämme münden, kann somit bei einer Platzierung der erfindungsgemäßen Pumpe 2 im Bereich des Venenwinkels der oberen Hohlvene auch die Wahrscheinlichkeit lymphogener Metastasierung verringert werden. Es ist aber auch denkbar mehrere Vorrichtungen gemäß der Erfindung an unterschiedlichen Stellen im venösen Abfluss VA eines vom Tumor betroffenen Organs oder Organteiles zu platzieren, um in deren Zusammenwirken den bestmöglichen Effekt zur Verringerung der Metastasenbildung zu erzielen.

## Patentansprüche

1. Vorrichtung zur Zerkleinerung oder Inaktivierung von zirkulierenden Tumorzellen (CTC) oder Tumorzellclustern (CTCC) aus einem von einem Tumor betroffenen Organ oder Organteil, **dadurch gekennzeichnet, dass** sie aus einer innerhalb eines äußeren Mantelrohres (9) angeordneten Pumpe (2) gebildet wird, die einen druckerhöhenden Abschnitt und eine am Pumpenausgang angeordnete druckreduzierende Drossel (13) aufweist, sowie ausgangsseitig in ihrem durch Volumenstrom (Q) und Pumpdruck (p) gegebenen Auslegungspunkt dem Volumenstrom und dem Blutdruck des venösen Abflusses (VA) des vom Tumor betroffenen Organs oder Organteils entspricht, wobei die Pumpe (2) als Mikroaxialpumpe mit einer entlang einer Schraubenwelle (6) der Mikroaxialpumpe angeordneten Abfolge von druckerhöhenden Schaufeln (12) ausgeführt ist, und die Drossel als ein radial von der Schraubenwelle (6) der Mikroaxialpumpe (2) abstehender Strömungswiderstand ausgeführt ist, der einen zylindrischen Abschnitt aufweist, der mit dem die Drossel (13) umhüllenden, äußeren Mantelrohr (9) einen kreisringzylindrischen Ringspalt (14) ausbildet.

2. Vorrichtung nach Anspruch 1, **dadurch gekennzeichnet, dass** der Volumenstrom (Q) der Pumpe (2) im Auslegungspunkt 0.1-1.5 Liter/Minute beträgt.

3. Vorrichtung nach Anspruch 1 oder 2, **dadurch gekennzeichnet, dass** der Pumpdruck (p) der Pumpe (2) im Auslegungspunkt 4-20 hPa beträgt.

4. Vorrichtung nach einem der Ansprüche 1 bis 3, **dadurch gekennzeichnet, dass** vor der Pumpe (2) und nach der Drossel (13) jeweils ein Drucksensor vorgesehen ist, sowie eine Regeleinheit, die die Druckdifferenz der von den beiden Drucksensoren gemessenen Druckwerte durch Variation der Drehzahl der Schraubenwelle (6) auf einen vorgegebenen Sollwert regelt.

5. Vorrichtung nach Anspruch 4, **dadurch gekennzeichnet, dass** der für die Druckdifferenz vorgegebene Sollwert 0 beträgt.

6. Vorrichtung nach einem der Ansprüche 1 bis 5, **dadurch gekennzeichnet, dass** innerhalb des äußeren Mantelrohres (9) ein koaxiales, inneres Mantelrohr (11) vorgesehen ist, das an den Schaufeln (12) der Mikroaxialpumpe befestigt ist.

## Claims

1. Device for comminution or inactivation of circulating tumor cells (CTC) or tumor cell clusters (CTCC) from a tumor-affected organ or organ part, **characterized in that** it is formed from a pump (2) arranged inside an outer jacket tube (9), which has a pressure-increasing section and a pressure-reducing throttle (13) arranged at the pump outlet, and corresponds on the output side in its design point given by volumetric flow (Q) and pumping pressure (p) to the volumetric flow and the blood pressure of the venous drain (VA) of the tumor-affected organ or organ part, wherein the pump (2) is designed as a micro-axial pump with a sequence of pressure-increasing blades (12) arranged along a screw shaft (6) of the micro-axial pump, and the throttle is designed as a flow resistance projecting radially from the screw shaft (6) of the micro-axial pump (2), which flow resistance has a cylindrical section which forms a circular-cylindrical annular gap (14) with the outer jacket tube (9) enclosing the throttle (13).

2. Device according to claim 1, **characterized in that** the volumetric flow (Q) of the pump (2) in the design point is 0.1-1.5 liters/minute.

3. Device according to claim 1 or 2, **characterized in that** the pumping pressure (p) of the pump (2) is 4-20 hPa in the design point.

4. Device according to one of the claims 1 to 3, **characterized in that** before the pump (2) and after the throttle (13) a respective pressure sensor is provided, and a control unit, which controls the pressure difference of the pressure values measured by the two pressure sensors to a predetermined setpoint value by varying the speed of the screw shaft (6).

5. Device according to claim 4, **characterized in that** the setpoint value predetermined for the pressure difference is 0.

6. Device according to one of the claims 1 to 5, **characterized in that** a coaxial, inner jacket tube (11) which is fixed to the blades (12) of the micro-axial pump is provided within the outer jacket tube (9).

## Revendications

1. Dispositif pour broyer ou inactiver des cellules tumorales circulantes (CTC) ou des clusters de cellules tumorales circulants (CTCC) provenant d'un organe ou d'une partie d'organe affectée par une tumeur, **caractérisé en ce qu'**il est formé par une pompe (2) disposée dans un tube d'enveloppe extérieur (9), qui présente un segment augmentant la pression et un étranglement (13) réduisant la pression disposé à la sortie de la pompe, et dont le point de dimensionnement défini par le débit volumique (Q) et la pression de pompage (p) correspond, à sa sortie, au débit volumique et à la pression sanguine du drainage veineux (VA) de l'organe ou de la partie d'organe affectée par la tumeur, la pompe (2) étant conformée comme une micropompe axiale avec une série de pales (12) augmentant la pression disposées le long d'un arbre porte-hélice (6) de la micropompe axiale et l'étranglement étant conformé comme un obstacle à l'écoulement qui dépasse dans le sens radial de l'arbre porte-hélice (6) de la micropompe axiale (2) et qui présente une partie cylindrique formant un espace annulaire (14) en forme de cylindre circulaire avec le tube d'enveloppe extérieur (9) qui entoure l'étranglement (13).

2. Dispositif selon la revendication 1, **caractérisé en ce que** le débit volumique (Q) de la pompe (2) est de 0,1 à 1,5 litre/minute au point de dimensionnement.

3. Dispositif selon la revendication 1 ou 2, **caractérisé en ce que** la pression de pompage (p) de la pompe (2) est de 4 à 20 hPa au point de dimensionnement.'

4. Dispositif selon l'une des revendications 1 à 3, **caractérisé en ce qu'**un capteur de pression est prévu en amont de la pompe (2) et un autre après l'étranglement (13), et il est prévu une unité de régulation qui régule la différence de pression entre les valeurs de pression mesurées par les deux capteurs de pression à une valeur de consigne prédéterminée en faisant varier la vitesse de rotation de l'arbre porte-hélice (6).

5. Dispositif selon la revendication 4, **caractérisé en ce que** la valeur de consigne prédéfinie pour la différence de pression est de 0.

6. Dispositif selon l'une des revendications 1 à 5, **caractérisé en ce qu'**est prévu à l'intérieur du tube d'enveloppe (9) extérieur un tube d'enveloppe intérieur (11) coaxial qui est fixé aux pales (12) de la micropompe axiale.
